# EUROPEAN PATENT APPLICATION

(11) **EP 2 017 246 A1**
(43) Date of publication of application: **21.01.2009**
(21) Application number: 07742816.7
(22) Date of filing: 02.05.2007
(51) Int. Cl.: C07C 5/27, C07B 61/00, C07C 13/615

(54) **METHOD FOR PRODUCTION OF ADAMANTANE**

(30) Priority: 10.05.2006 JP 2006131273
(71) Applicant: Idemitsu Kosan Co., Ltd., Chiyoda-ku Tokyo 100-8321 (JP)
(72) Inventor: SAITO, Masao, Ichihara-shi Chiba 299-0193 (JP); KOJIMA, Akio, Ichihara-shi Chiba 299-0193 (JP); MASE, Jun, Ichihara-shi Chiba 299-0193 (JP); KUSABA, Toshiaki, Ichihara-shi Chiba 299-0193 (JP)
(74) Representative: Vossius & Partner
(86) International application number: PCT/JP2007/059381
(87) International publication number: WO 2007/129665

(57) **Abstract**

Provided is a method for production of adamantanes, in which, when a tricyclic saturated hydrocarbon compound having 10 or more carbon atoms is isomerized in the presence of a solid catalyst to give adamantanes, no solid matter derived from an oxygen-containing compound is formed or accumulated in the inlet or the bottom of an isomerization reactor by providing a pre-reactor in an isomerization step even if the oxygen-containing compound is present in a raw material to be supplied.

## Description

### Technical Field

The present invention relates to a method for production of adamantanes, which involves using a pre-reactor in an isomerization process. More specifically, the present invention relates to a method of continuously producing high-purity adamantanes using a solid catalyst, in which a pre-reactor is provided in an isomerization process so that a long-term continuous operation thereof is achieved.

### Background Art

Adamantanes is a stable, highly symmetrical compound in which four cyclohexane rings are bonded to form a cage-like structure. It has been known that adamantanes, which has such a specific adamantane skeleton and shows peculiar functions, is useful for raw materials or intermediates of various chemicals such as lubricants, agricultural and medical raw materials, and highly functional industrial materials.
In general, there have been known a method for production of adamantanes including isomerizing a tricyclic saturated hydrocarbon compound having 10 or more carbon atoms as a raw material (for example, refer to Patent Document 1).
Aluminum halides have been conventionally known as isomerization catalysts for isomerizing the tricyclic saturated hydrocarbon compound having 10 or more carbon atoms as a rawmaterial. In addition, as a solid catalyst, there has been known a catalyst in which an active metal such as platinum or rhenium is supported by a cation-exchanged zeolite with an impregnation method (for example, refer to Patent Document 2). In addition, there has been also known a catalyst in which one kind or two or more kinds of metals selected from the groups 8 to 10 of the periodic table is supported by a zeolite with an ion-exchange method (for example, refer to Patent Document 3).
In general, the isomerization is performed by supplying the tricyclic saturated hydrocarbon compound having 10 or more carbon atoms as a raw material in an isomerization reactor filled with the solid catalyst in conditions where the temperature is about 300 to 500°C and the pressure is about normal pressure to 20 MPa. However, if an oxygenated compound is present in the raw material to be supplied, a solid matter derived from the oxygen-containing compound is formed and is accumulated in the inlet or on the bottom of the reactor. As a result, there occurs a problem that the differential pressure inside the reactor is increased and the reactor should be shut down urgently.

Patent Document 1: JP 2004-59483 A
Patent Document 2: JP 52-2909 B
Patent Document 3: JP 2004-51484 A

### Disclosure of the Invention

### Problems to be solved by the Invention

The present invention has been made with a view to solving the problem under the above-mentioned circumstances. An object of the present invention is to provide a method for production of adamantanes, in which, when the tricyclic saturated hydrocarbon compound having 10 or more carbon atoms is isomerized in the presence of the solid catalyst to give the adamantanes, no solidmatter derived from the oxygen-containing compound is formed or is accumulated in the inlet or on the bottom of the isomerization reactor in the isomerization step even if the raw material to be supplied contains the oxygen-containing compound.

### Means for solving the Problems

The inventors of the present invention studied extensively to achieve the above object. As a result, the inventors found that, by providing a pre-reactor prior to an isomerization reactor in an isomerization step, adamantanes could be continuously produced stably for a long time without formation or accumulation of a solid matter derived from an oxygen-containing compound in the inlet or on the bottom of the isomerization reactor even if the oxygen-containing compound is present in a raw material to be supplied. Then, the inventors completed the present invention based on these findings.

That is, the present invention provides:
(1) a method for production of adamantanes including isomerizing a tricyclic saturated hydrocarbon compound having 10 or more carbon atoms in a presence of a solid catalyst, in which a pre-reactor is provided in an isomerization process;
(2) the method for production of adamantanes according to item (1), in which the pre-reactor is filled with the same solid catalyst as a solid catalyst filled in an isomerization reactor;
(3) the method for production of adamantanes according to item (1) or (2), further including continuously supplying the tricyclic saturated hydrocarbon compound having 10 or more carbon atoms to the pre-reactor in an upflow direction;
(4) the method for production of adamantanes according to any one of items (1) to (3), in which the pre-reactor is operated at temperature of 120 to 180°C; and
(5) the method for production of adamantanes according to any one of items (1) to (4), in which the tricyclic saturated hydrocarbon compound having 10 or more carbon atoms includes trimethylene norbornane.

### Effects of the Invention

According to the present invention, a method for production of adamantanes including isomerizing the tricyclic saturated hydrocarbon compound having 10 or more carbon atoms in the presence of the solid catalyst, in which no solid matter derived from the oxygen-containing compound is formed or accumulated in the inlet or on the bottom of the isomerization reactor in the isomerization step even if the oxygen-containing compound is present in the raw material to be supplied can be provided.

### Brief Description of the Drawings

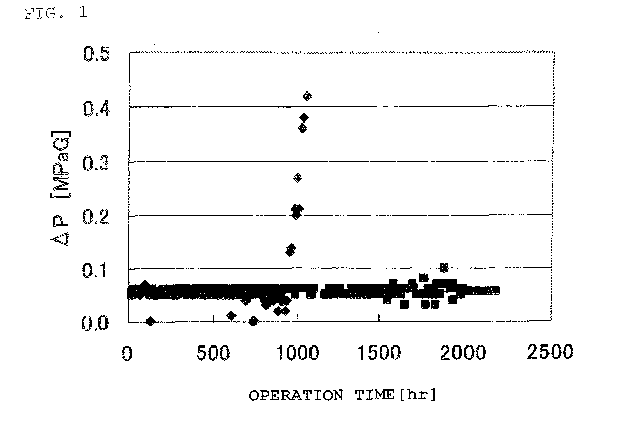

FIG. 1 is a view showing an effect of a reaction time on a differential pressure.

### Description of Symbols

□: with pre-reactor
◇: without pre-reactor

### Best Mode for carrying out the Invention

The method for production of adamantanes of the present invention is characterized in that a pre-reactor is provided in an isomerization step when a tricyclic saturated hydrocarbon compound having 10 or more carbon atoms is isomerized in the presence of a solid catalyst to give adamantanes.

A method for production of adamantanes by isomerizing a tricyclic saturated hydrocarbon compound having 10 or more carbon atoms as a raw material generally includes (A) an isomerization step of isomerizing a raw material, (B) a concentration step of concentrating adamantanes contained in the resultant reaction liquid, (C) a crystallization step of precipitating the concentrated adamantane, (D) a solid-liquid separation step of separating the adamantane crystals from the crystallized slurry solution, (E) a washing step of washing the adamantane crystals separated from the liquid, and (F) a drying step of drying the washed adamantane crystals.

### Hereinafter, each step is described.

### (A) Isomerization step

This reaction step includes supplying a raw material in an isomerization reactor filled with a solid catalyst with a batch mode or a continuous mode and isomerizing the raw material to give adamantanes. As the raw material, a tricyclic saturated hydrocarbon compound having 10 or more carbon atoms is used. A tricyclic saturated hydrocarbon compound having 10 to 15 carbon atoms is preferred as the tricyclic saturated hydrocarbon compound having 10 or more carbon atoms. Examples thereof include trimethylene norbornane (TMN, tetrahydrodicyclopentadiene), perhydroacenaphthane, perhydrofluorene, perhydrophenalene, 1,2-cyclopentanoperhydronaphthaline, perhydroanthracene, and perhydrophenanthrene. Further, alkyl substituents of those compounds such as 9-methyl perhydroanthracene is preferably exemplified. The saturated hydrocarbon compound having 10 or more carbon atoms can be easily obtained by hydrogenating the tricyclic unsaturated hydrocarbon compound having 10 or more carbon atoms such as dicyclopentadiene, acenaphthene, fluorene, phenalene, 1,2-cyclopentanonaphthaline, anthracene, phenanthrene, or 9-methyl anthracene by using a catalyst for hydrogenation.

The solid catalyst used in the isomerization of the tricyclic hydrocarbon compound having 10 or more carbon atoms is not particularly limited, and a solid acid catalyst, in particular, a metal-supporting solid acid catalyst is preferred.
The kind of metal in the metal-supporting solid acid catalyst is not particularly limited as long as the metal has such an active function that the tricyclic saturated hydrocarbon compound having 10 or more carbon atoms is isomerized to adamantanes, and various metals may be used. Examples of the active metal include rare earth metals, alkali earth metals, metals belonging to the groups 8 to 10 of the periodic table, and rhenium. Specific examples thereof include iron, cobalt, nickel, ruthenium, rhodium, palladium, rhenium, osmium, iridium, and platinum. One kind of the active metal may be supported alone, or two or more kinds of active metals may be supported in combination. Of those, a solid acid catalyst supporting platinum is particularly preferred.
The supporting amount of those active metals is not particularly limited but is generally about 0.1 to 5 mass% based on the total catalyst amount from the viewpoint of catalyst activity.

The solid acid supporting any one of those metals is not particularly limited, and various solid acids including various zeolites such as an A-type zeolite, an L-type zeolite, an X-type zeolite, a Y-type zeolite, zeolite β, and ZSM-5, silica-alumina, alumina, heteropolyacids, and sulfated zirconia are exemplified. Of those, metal oxides of the A-type zeolite, L-type zeolite, X-type zeolite, Y-type zeolite, zeolite β, and ZSM-5, silica-alumina, alumina, heteropolyphosphoric acids, and sulfated zirconia are preferred. Of those solid acids, the X-type zeolite and Y-type zeolite are particularly preferred.

In addition, a metal-supporting solid acid catalyst can be produced by causing the zeolite serving as a carrier to support at least one kind of the above metals by an ion exchange method or an impregnation method.
Here, when the catalyst is produced by an ion exchange method, the catalyst can be obtained by: bringing a metal salt of the above metals or a metal complex salt aqueous solution of the above metals into contact with zeolite; subjecting a cation site (such as H⁻ or NH₄⁺) in zeolite to ion exchange; drying the resultant; and calcining the resultant.
In addition, in the case of the impregnation method, the catalyst can be obtained by: mixing zeolite with the metal salt of the above metals or the metal complex salt aqueous solution of the above metals; drying the mixture by evaporation using a rotary evaporator or the like to give a solid; and impregnating the resultant with the metal to cause the resultant to support metal.
In the case of producing the metal-supporting acid catalyst by using the sulfated zirconia as a carrier, the catalyst can be obtained by causing at least one kind of the metals to be supported by the sulfated zirconia by the impregnation method.
The catalyst thus obtained may be one of a powdery form and a granular form. In addition, the shape of the reactor used in the isomerization is not particularly limited. For example, ass-called fixed bed continuous reactor used for filling the catalyst and supplying a raw material continuously may be used, but the reactor is not limited thereto. In addition, the reactor may be a continuous mode or a batch mode. In addition, all styles such as a general solid-liquid contact type or a general solid-gas contact type may be used.

In the isomerization, the tricyclic saturated hydrocarbon compound having 10 or more carbon atoms may be used in the reaction after purification, or used directly without purification.
In both cases, the compound can be used together with a solvent. In this case, the solvent ratio is generally about 0 to 10 parts by mass and preferably 0 to 3 parts by mass with respect to 1 part by mass of the tricyclic saturated hydrocarbon compound having 10 or more carbon atoms. When the tricyclic saturated hydrocarbon compound having 10 or more carbon atoms is used directly without purification, the solvent ratio can be adjusted to the above ratio by removing part of the solvent used in the hydrogenation step or adding additional solvent.
As operation conditions of the isomerization reactor, the reaction temperature is generally about 200 to 500°C, and preferably 250 to 400°C, and the pressure is generally about normal pressure to 20 MPa, and preferably 2 to 8 MPa, and the reaction is preferably performed in the presence of hydrogen from the viewpoint of improving the yield.
As the reaction style, any one of a circulation mode and a batch mode can be applied, and the circulation mode is preferred. In the case of the circulation mode, a mass hourly space velocity (MHSV) is generally 0.01 to 20 h⁻¹, and preferably selected from the range of 0.1 to 10 h⁻¹, and a molar ratio of hydrogen to raw material is generally 0.1 to 10, and preferably selected from the range of 1 to 5.
On the other hand, in the case of the batch mode, a catalyst amount is generally 0.01 to 2, and preferably selected from the range of 0.05 to 1 in a mass ratio of catalyst to raw material. In addition, the reaction time is generally about 1 to 50 hours.

The present invention is characterized in that, in the isomerization step, the pre-reactor is arranged prior to the isomerization reactor in series. The pre-reactor is also filled with the same isomerization catalyst as that in the isomerization reactor. Operation conditions of the pre-reactor are the same as those of the isomerization reactor except that an inlet temperature and an outlet temperature of the reactor are controlled to about 120 to 150°C and about 160 to 180°C, respectively. The tricyclic saturated hydrocarbon compound having 10 or more carbon atoms as the material for the isomerization generally contains about 500 to 1, 000 mg/L of oxygen derived from the oxygen-containing compound. When the raw material is supplied, as it is, to the regular isomerization reactor, the oxygen-containing compoundbecomes heavy to generate a solid matter because the reaction temperature is high. The solid matter is accumulated in the inlet or on the bottom of the isomerization reactor, with the result that the differential pressure inside the reactor is increased and the reactor is forced to stop the operation.
In the present invention, in order to prevent the formation or accumulation of the solid matter due to the oxygen-containing compound, the pre-reactor is provided prior to the isomerization reactor, whereby the oxygen-containing compound in the raw material is treated inside the pre-reactor at relatively low temperature prior to supplying the raw material to the isomerization reactor. Therefore, the oxygen amount derived from the oxygen-containing compound in the raw material at the outlet of the pre-reactor is decreased to about 100 to 200 mg/L, whereby it is suppressed that the oxygen-containing compound becomes heavy-duty material in the isomerization reactor, and the formation and accumulation of the solidmatter is prevented. As a result, the present invention enables stable operation in the isomerization step and stable operation in the whole production process for adamantanes.

In the present invention, it is preferred that the oxygen-containing compound in the raw material be treated by continuously supplying the tricyclic saturated hydrocarbon compound having 10 or more carbon atoms as a raw material to the pre-reactor in an upf low direction. In order to supply the compound continuously, a so-called fixed bed continuous reactor filled with a catalyst is preferably used. As the reaction style, any one of a solid-liquid contact type and a solid-gas contact type can be used.
The continuous supplying of the raw material in the upflow direction to the pre-reactor is preferred because liquid phase (tricyclic saturated hydrocarbon compound having 10 or more carbon atoms as a raw material) becomes a continuous phase, and hydrogen is diffused in the liquid to reach the catalyst surface, whereby the oxygen-containing compound can be treated efficiently.

The oxygen-containing compound in the tricyclic saturated hydrocarbon compound having 10 or more carbon atoms as a raw material is confirmed to be 2,5-dimethyl tetrahydrofuran, benzaldehyde, acetophenone, p-triyl aldehyde, o-triyl aldehyde, n-dibutyl ether, 1-indanone, p-methyl acetophenone, m-methyl acetophenone, and 2-cyclopentyl cyclopentanone.

In the present invention, in the tricyclic saturated hydrocarbon compound having 10 or more carbon atoms as a raw material for the isomerization, a monocyclic saturated hydrocarbon compound, an aromatic compound, and water may coexist. Here, as the monocyclic saturated hydrocarbon compound to be coexisted, cyclopentane, cyclohexane, ethylcyclohexane, methylcyclohexane, and the like are exemplified.
Cyclohexane or ethylcyclohexane, or a mixture of them is particularly preferred.
In addition, examples of the aromatic compound include: aromatic hydrocarbon compounds such as benzene, toluene, xylene, naphthalene, and anthracene; nitrogen-containing aromatic compounds such as aniline and nitrobenzen; and halogen-containing aromatic compounds such as chlorbenzene and bromobenzene. Of those aromatic compounds, the aromatic hydrocarbon compounds such as benzene, toluene, xylene, naphthalene, and anthracene are more preferred, and benzene is particularly preferred. The addition amount of each of those compounds to be coexisted is not particularly limited, and can be appropriately selected depending on various situations.

### (B) Concentration Step

The concentration step is a step of subjecting the resultant isomerization liquid obtained in the above isomerization step to a concentration treatment with a single flash column or distillation column, or a combination of multiple flash columns and distillation columns according to a batch mode or a continuous mode so that the solvent and a light by-product (impurity) are removed, and the liquid is concentrated up to the concentration at which the liquid can be efficiently crystallized in the subsequent crystallization step. In the concentration step, an unreacted light gas such as hydrogen is removed by using the flash column, and the concentration is completed by using one distillation column in ordinary cases. In addition, the concentration is performed in such a manner that adamantanes concentration becomes ordinarily 10 to 50 mass%, or preferably 20 to 40 mass%.
When the concentration ratio is excessively low, the recovery efficiency of the adamantanes in the crystallization step degrades. On the other hand, when the concentration ratio is excessively high, impurities are also relatively concentrated, so the impurities are apt to be taken in the adamantanes in the crystallization step.
The distillation column is not particularly limited, and generally, a distillation column used for distillation can be used as it is, and may be a packed distillation column or a multistage tray distillation column. A multistage tray distillation column having about 10 to 20 stages is preferred. As operation conditions of the distillation column, the temperature of the column bottom is about 110 to 200°C, and the pressure inside the column is about
- 100 kPa to normal pressure, whereby the liquid is concentrated to the adamantane concentration above.

Part or all of the light fraction obtained from overhead of the distillation column is used as a solvent in the isomerization step or hydrogenation step in certain cases, and hence additional use amount of the reaction solvent can be suppressed to the minimum, and a raw material and a reaction intermediate fraction contained in the light fraction or the adamantanes can be used by recycle. In addition, part or all of the overhead liquid is used as a recrystallization solvent in the subsequent step, that is, crystallization step, and hence an additional amount of the recrystallization solvent can be suppressed to the minimum.
The recycle amount for the isomerization step or the use amount of the recrystallization solvent is appropriately adjusted by the adamantane concentration in the fraction obtained from the overhead in the distillation column or the concentrations of other impurities, or a flow balance in the process. As a result, a product with an intended purity can be obtained efficiently.

### (C) Crystallization Step

The crystallization step is a step of crystallizing the adamantanes from the concentrate obtained in the above concentration step according to a batch mode or a continuous mode.
General cooling crystallization or evaporation crystallization, or a combination of them can be employed as a crystallization operation. The operation temperature in the crystallization operation depends on the adamantane concentration of the concentrate. In the case of continuous crystallization, the operation temperature is ordinarily about -20 to 50°C, or preferably 0 to 30°C. When the temperature is lower than -20°C, a large amount of energy for cooling is consumed. When the temperature exceeds 50°C or higher, the solubility of the adamantanes in a solvent is so high that the recovery efficiency of the adamantanes may decrease. In addition, in any other crystallization methods, it is advantageous to define the temperature at which the solubility of adamantane is about 0.5 to 25 mass%, or preferably about 5 to 15 mass % as the final temperature in the crystallization step because of the same reason. When an impurity causing a problem in terms of quality is taken in the concentrate by one crystallization, recrystallization may be performed immediately after the crystallization. Alternatively, after a solid-liquid separation step and a washing step as post-processing steps, the recrystallization, the solid-liquid separation step, and the washing step may also be repeated multiple times.

### (D) Solid-Liquid Separation Step

The solid-liquid separation step is a step of separating the adamantane crystals precipitated in the crystallization step and the solvent according to a batch mode or a continuous mode. As the solid-liquid separation operation, a general method using a filter cloth, a sintered metal, or the like may be used. In this case, part of the separated mother liquid is generally exhausted outside the system in order to suppress the concentration of impurities. Then, by recycling part or all of the remainder in the concentration step or crystallization step, loss of the adamantanes can be suppressed to the minimum. Alternatively, by using part or all of the remainder as part of the solvent or raw material in the isomerization step, or the hydrogenation step in certain cases, novel solvent is not needed, or a raw material and a reaction intermediate fraction contained in the light fraction, or the adamantanes can be recycled and used. The solid-liquid separation is performed in such a degree that the liquid content in the separated crystal cake is preferably about 50 mass% or less, and more preferably 5 to 30 mass%. The exhaust amount of the mother liquid to outside the system or the recycle amount of the mother liquid is appropriately adjusted by the concentration of the adamantanes contained in the mother liquid or the concentration of the other impurities, or flow balance in the process, whereby a product with an intended purity can be obtained efficiently.

### (E) Washing Step

The washing step is a step of washing and removing the solvent that has not been sufficiently removed in the above solid-liquid separation by using a washing solvent. As the washing solvent, almost all organic solvents can be used, but polar solvents such as alcohols, ketones, carboxylic acids each having low solubility are preferred in order not to decrease the recovering efficiency of the adamantanes .
In addition, in the case where the washed adamantane is directly treated in the subsequent step, that is, the drying step, a solvent having a low boiling point, which is easily dried, is preferred. In general, a solvent having a boiling point of 150°C or lower, and, in particular, a solvent having a boiling point of about 30 to 100°C is suitable.
Examples of the solvent include methanol, ethanol, propanol, isopropanol, acetone, methyl ethyl ketone, diethyl ketone, diethyl ether, acetic acid, and carbon tetrachloride.
The washing operation temperature ordinarily is in the range of room temperature to a temperature equal to or lower than the boiling point of the washing solvent, or preferably -20 to 50°C.

### (F) Drying Step

The drying step is a step of subjecting the washed adamantane crystals obtained in the above washing step to drying treatment. A general drying machine that has been industrially used, such as a reduced-pressure-type drying machine or a heating-type drying machine, can be used in the drying treatment. In addition, a drying method may follow one of a continuous mode and a batch mode.
Because the object of the drying treatment lies in the removal of the washing solvent, the operating conditions for the treatment vary depending on the kind of a solvent to be used in the washing step. In ordinary cases, the pressure is equal to or lower than normal pressure, or preferably 5 to 101 kPa, and the temperature is equal to or lower than the boiling point of the washing solvent, or preferably 20 to 60°C.
Thus, no troublesome operation such as waste liquid disposal is needed, and high-purity adamantane can be efficiently produced while the loss thereof is minimized.

### Examples

Next, the present invention is described in more detail by way of examples. However, the present invention is not limited to those examples.

### Preparation Example 1

### Preparation of metal-supporting solid acid catalyst

1.275 kg of Y-type zeolite subjected to sodium ion-exchange (hereinafter, referred to as NaY) was suspended while being stirred in 7 kg of pure water, and the mixture was heated to 60°C. While the mixture was stirred, 8 kg of an aqueous solution of mixed rare earth chlorides (mixed chlorides containing rare earth metals at a ratio of 49 mass% of Ce, 24 mass% of La, 20 mass% of Nd, 5 mass% of Pr, and 2 mass% of Sm) (containing in terms of 890 g of (RE₂O₃ (CeO₂ + La₂O₃ + Nd₂O₃ + Pr₂O₃ + Sm₂O₃) in total) was added to the mixture, followed by stirring for 2 hours. After the powder was filtrated, the resultant was washed with 15 kg of pure water, and calcined at 650°C for 3 hours after drying (primary exchange with mixed rare earth) . After the calcination, 340 g of powders were suspended in 2 kg of warm water at 60°C. While the obtained mixture was stirred, hydrochloric acid was added to the mixture until the pH of the mixture reached 5.01. To the slurry thus obtained, added were 2 kg of the aqueous solution of the mixed rare earth (containing 130.6 g in terms of RE₂O₃ (CeO₂ + La₂O₃ + Nd₂O₃ +Pr₂O₃ + Sm₂O₃) in total), and the resultant was stirred at 60°C for 2 hours. The powders were recovered by filtration, and then washed with 4 kg of pure water (secondary exchange with rare earth). 400 g of the thus obtained powders were resuspended in 2 kg of pure water, and 722 g of 1.0 mass% aqueous solution of tetramine platinum chloride were added to the mixture, followed by stirring at 30°C for 2 hours. The resultant was filtered and washed, and dried at 110°C overnight, whereby Re-containing Y-type zeolite supporting 1.0 mass% of platinum was obtained.

### Example 1

### (1) Isomerization step

A stainless steel pre-reactor tube having a full length of 60 cm and an aperture of 14 mm and an isomerization reactor tube were each filled with 13 g of the catalyst obtained in Preparation Example 1 above. In addition, the catalyst was calcined at 300°C for 3 hours in a stream of air. After the air in the tubes were substituted with nitrogen, the resultant was subjected to hydrogen reduction at 300°C for 2 hours under the normal pressure and in a stream of hydrogen.
After that, a mixed solution including decalin and trimethylene norbornane (TMN) containing 1,000 mg/L of oxygen derived from an oxygen-containing compound at a mass ratio of 2 : 1 was supplied to the pre-reactor tube from the bottom thereof in an amount of 32 ml/hr, and hydrogen was also supplied to the pre-reactor from the bottom thereof. Apre-treatment was performed in the conditions where the pressure was 6 MPa, the inlet temperature and the outlet temperature of the pre-reactor tube were 150°C and 175°C, respectively, the mass hourly space velocity (MHSV) was 2.4 h⁻¹, and the ratio of hydrogen to TMN was 2.0, whereby an oxygen amount in the outlet, which was derived from the oxygen-containing compound, was decreased to 100 mg/L. Next, the mixture was passed to the isomerization reactor tube in which the inlet temperature of the reactor tube was set to 325°C, and then the mixture was isomerized continuously for 3 months. It was not confirmed that a solid matter due to the oxygen-containing compound was formed or accumulated in the inlet or on the bottom of the isomerization reactor tube. In addition, the increase of the differential pressure in the isomerization reactor tube was not confirmed and, stable, continuous operation was performed.

### Comparative Example 1

An isomerization was performed in accordance with Example 1 except that the pre-reactor tube was not used. After 1 month, it was confirmed that a solid matter was formed in the isomerization reactor tube. Then, the differential pressure was increased, with the result that the operation was stopped urgently.
Note that FIG. 1 shows the relationship between the operation time and the differential pressure in Example 1 and Comparative Example 1.

### Industrial Applicability

The present invention provides a method for production of adamantanes, in which, when a tricyclic saturated hydrocarbon compound having 10 or more carbon atoms is isomerized in the presence of a solid catalyst to give adamantanes, no solid matter derived from an oxygen-containing compound is formed or accumulated in the inlet or on the bottom of the isomerization reactor in the isomerization step even if the oxygen-containing compound is present in a raw material to be supplied.

## Claims

1. A method for production of adamantanes, comprising isomerizing a tricyclic saturated hydrocarbon compound having 10 or more carbon atoms in a presence of a solid catalyst, wherein a pre-reactor is provided in an isomerization process.

2. The method for production of adamantanes according to claim 1, wherein the pre-reactor is filled with the same solid catalyst as a solid catalyst filled in an isomerization reactor.

3. The method for production of adamantanes according to claim 1, further comprising continuously supplying the tricyclic saturated hydrocarbon compound having 10 or more carbon atoms to the pre-reactor in an upflow direction.

4. The method for production of adamantanes according to claim 1, wherein the pre-reactor is operated at temperature of 120 to 180°C.

5. The method for production of adamantanes according to claim 1, wherein the tricyclic saturated hydrocarbon compound having 10 or more carbon atoms comprises trimethylene norbornane.
